# EUROPEAN PATENT SPECIFICATION

(11) **EP 2 101 661 B1**
(45) Date of publication and mention of the grant of the patent: **02.03.2016**
(21) Application number: 07868030.3
(22) Date of filing: 26.12.2007
(51) Int. Cl.: A61F 2/95, A61M 25/06

(54) **DELIVERY SYSTEM AND SHEATH FOR ENDOLUMINAL PROSTHESIS**
TRÄGERSYSTEM UND SCHLEUSE FÜR EINE ENDOLUMINALE PROTHESE
SYSTÈME DE DÉLIVRANCE ET GAINE POUR PROTHÈSE ENDOLUMINALE

(30) Priority: 26.12.2006 US 877201 P
(43) Date of publication of application: 23.09.2009
(73) Proprietor: Cook Medical Technologies LLC, Bloomington, IN 47404 (US)
(72) Inventor: POULSEN, Fleming, 2400 Koebenhavn NV (DK)
(74) Representative: Garratt, Peter Douglas
(86) International application number: PCT/US2007/026317
(87) International publication number: WO 2008/085470

(56) References cited:
- EP-A- 0 696 447
- EP-A- 0 749 731
- EP-A- 1 637 176
- WO-A-01/34061
- US-A- 5 026 377
- US-A1- 2003 236 545

## Description

### Technical Field

This invention relates generally to medical devices and, in particular, to a delivery system for stents and other endoluminal prostheses.

### Background of the Invention

Stents are commonly used to treat stenosis of various arteries. Where blood vessels are clogged or narrowed by substances that restrict blood flow, stents are delivered into such vessels and expanded to dilate blood vessels or maintain the dilated state of blood vessels. Expansion of stents may be made with or without the aid of a balloon. Balloon-expandable stents are expanded by inflating a balloon disposed beneath the stent. On the other hand, self-expandable stents are capable of expanding without the use of a balloon. For this purpose, self-expandable stents are generally made from shape memory or spring metal, such as Nitinol or stainless steel, so that self-expandable stents are able to expand from a compressed state upon removal of pressures applied thereon.

Delivery catheters or sheaths are widely used for delivering a stent or a stent graft to a deployment site well within the vasculature of the patient. A delivery sheath as defined in the preamble of claim 1 is disclosed in US2003/236545.

Typically, the delivery catheter is inserted over a guide wire. In one common arrangement, used particularly with self-expanding stents, an inner core carries the stent and has a distal tip that is atraumatic and may assist in dilating the vessel as the delivery catheter advances along the guide wire. A sheath covers the stent during the delivery procedure and maintains or assists in maintaining the stent in its radially compressed configuration. The distal tip will usually have a smooth outer surface, tapering axially from a relatively large outer diameter, corresponding to the outside diameter of the sheath, to a relatively small outer diameter at the distal end of the sheath, corresponding (with an appropriate wall thickness for the tip) to the outside diameter of the wire guide. Once the stent has reached the deployment site, the sheath is withdrawn, uncovering the stent and allowing it to expand radially.

Once the stent has been deployed, the inner core - together with its distal tip -can be withdrawn. The largest diameter of the distal tip is usually larger than the outside diameter of the stent in its compressed form. However, provided that the inner diameter of the radially expanded stent is sufficiently greater than the largest outside diameter of the distal tip, the distal tip of the inner core can now pass through the stent and, with appropriate care, there is no significant risk of the withdrawal of the tip dislodging or otherwise interfering with the deployed stent.

It will be recognized that the tip carried at the distal end of the inner core has the important function of providing an atraumatic surface positioned forwardly of the distal end of the stent as the stent is advanced through the vasculature. This surface may assist passage through a stenosis. In some applications, the atraumatic surface may also serve a dilating function. Without such a surface positioned forwardly of the stent, there would be a risk of the distal end of the stent engaging the vessel wall, causing damage or preventing forward movement of the delivery sheath.

It is however not always appropriate or possible to have a distal tip carried on the inner core. With, for example, stents of smaller radius it is not always possible or convenient to provide a tip that is larger in diameter than the outer diameter of the expanded stent yet sufficiently smaller in diameter than the inner diameter of the expanded stent to be withdrawn safely and reliably through the stent after deployment. This problem is exacerbated with longer stents that may be deployed over curved sections of a vessel.

It has accordingly been proposed to provide a stent delivery sheath in which there is no distal tip carried on the inner core. Instead, the sheath that covers the stent during delivery and is retracted relative to the stent during deployment, has at its distal end a portion which is formed of an elastic material which can be resiliently deformed radially outwardly to accommodate the stent but which, forwardly of the stent, relaxes to a narrower diameter which is less than the diameter of the stent and approaches the outside diameter of the wire guide. As the delivery sheath is advanced forwardly through the vasculature, the elastic portion of the sheath provides the required atraumatic or dilating surface positioned forwardly of the stent. Once the stent has reached the deployment site, the sheath is retracted relative to the stent and the distal portion that was forwardly of the distal end of the stent stretches radially outwardly to allow it to pass over the stent.

The absence of a distal tip that is require to pass through the stent after deployment, may offer a number of important advantages. The risk is removed of a retracting distal tip catching the inner surface of the deployed stent. The fact that, during deployment, there is no distal tip as such past the stenosis, may be advantageous in small vessels. In some cases, it may be an advantage in manufacture of the device, that there is no distal tip to be added as a final step to the inner core of the device.

It is clearly desirable for the distal portion of the sheath, which stretches and slides over the sheath on deployment, to have both good elastic properties and a low coefficient of friction in its sliding contact with the stent. Unfortunately, materials such as PTFE that are customarily employed to provide high lubricity or low friction, do not exhibit good elasticity. Similarly, materials such as Nylon or PEBAX, which have the required elasticity, do not offer high lubricity.

One attempt to solve this problem is disclosed in US 2002/0183826. In the delivery catheter there disclosed, a thermoplastic polymer sheath has a PTFE lining to provide the required lubricity. To ensure that the distal tip of the sheath is sufficiently elastic, the PTFE lining stops short of the distal end of the sheath.

It is found however that even with this arrangement there remains the risk that the distal end of the sheath may catch the stent on retraction relative to the stent, especially if the stent at deployment is located in a curved part of the vascular system. This can cause misalignment of the stent and also harm the vascular system.

### Summary of the invention

The present invention consists in one aspect in a delivery sheath for delivering stents or other endoluminal prostheses, the sheath having a distal end with a radially inner sheath layer and a radially outer sheath layer both extending to the distal end of the sheath, wherein the radially inner layer comprises a material having a low coefficient of friction and the radially outer layer comprises an elastic material; and wherein the radially inner layer has at least one separation line extending longitudinally of the sheath to the distal end to define at least two leaf edges in the radially inner layer which are configured to move circumferentially with respect to each other to accommodate elastic radial expansion of the overlying radially outer layer.

Preferably, the radially inner layer has at least two separation lines extending longitudinally of the sheath to the distal end to define at least two leaves which are configured to move circumferentially with respect to each other to accommodate elastic radial expansion of the overlying radially outer layer. The separation line (or each separation line) may be a slit or a line of weakness in the radially inner layer. One leaf edge may overlie another leaf edge so that the leaf edges move circumferentially toward each other to accommodate elastic radial expansion of the overlying radially outer layer. The outer diameter of the delivery sheath may taper with the diameter reducing in a direction toward the distal end. The resistance of the outer layer to hoop stresses may decrease towards the distal end.

In another aspect, the present invention consists in a delivery system comprising a delivery sheath having a distal portion at a distal end; an endoluminal prosthesis located within the delivery sheath in the distal portion so that the distal portion of the delivery sheath extends circumferentially around the prosthesis, the delivery sheath tapering distally of the prosthesis to a reduced diameter at the distal end of the sheath so providing a dilating surface distally of the prosthesis, the distal portion of the delivery sheath comprising a radially inner sheath layer and a radially outer sheath layer both extending to the distal end of the sheath, wherein the radially inner layer comprises a material having a low coefficient of friction and the radially outer layer comprises an elastic material; and wherein the radially inner layer has at least one separation line extending longitudinally of the sheath to the distal end to define at least two leaf edges in the radially inner layer which are configured to move circumferentially with respect to each other to accommodate elastic radial expansion of the overlying radially outer layer as the sheath is retracted proximally with respect to the prosthesis.

### Brief Description of the Drawing

The invention will now be described by way of example, with reference to the accompanying drawings, in which:
Figure 1 is a somewhat diagrammatic longitudinal section through a stent delivery system, according to one example;
Figure 2 is a radial section through the stent delivery system on line 2-2 of Figure 1;
Figure 3 is a perspective diagram of the stent delivery system shown in Figures 1 and 2;
Figure 4 is a view similar to Figure 1 but showing an initial stage in stent deployment;
Figure 5 is a radial section through the stent delivery system on line 5-5 of Figure 4;
Figure 6 is a view similar to Figure 1, illustrating a modification;
Figure 7 is a radial section through the stent delivery system on line 7-7 of Figure 6; and
Figure 8 is a view similar to Figure 5, of the stent delivery system shown in Figures 6 and 7.

### Detailed Description

Determining the proper stent to use is the first step to deploying a stent. An appropriate stent delivery mechanism is then identified. This will depend - among other things - upon the nature of the stent, the vessel in which it is to deployed, and the purpose to be served by the stent.

Delivery systems for self expanding stents often include an atraumatic or dilating surface positioned distally of the stent as the stent is advanced through the vasculature (or other delivery route). Often this surface will be provided on a relatively rigid distal tip which is positioned distally of the stent during delivery and which is withdrawn proximally through the stent, once the stent has been deployed in its radially expanded configuration. In other cases, such a distal tip is inappropriate and the distal surface is required to be provided on a sheath which surrounds the stent and which is withdrawn proximally over the stent, during deployment. The distal portion of such a sheath is preferably configured so as optimally to provide:
▪ an effective atraumatic or dilating surface positioned distally of the stent during delivery;
▪ appropriate elasticity to enable the sheath - and particularly that portion of the sheath which provided the distal surface - to be readily retracted proximally over the stent; and
▪ a reduced-friction interior surface which faces the stent and which minimizes the risk of any interaction between the sheath and the stent which dislodges the stent or hampers such retraction.

In one example of the present invention, a stent or other endoluminal prosthesis is located within a delivery sheath that tapers distally of the stent so providing an atraumatic or dilating surface distally of the prosthesis. The distal portion of the delivery sheath comprising a radially inner sheath layer and a radially outer sheath layer both extending to the distal end of the sheath. The radially inner layer comprises PTFE or other material having a low coefficient of friction and the radially outer layer comprises an elastic material such as soft polymer with high flexibility. The radially inner layer has at least one slit or separation line extending longitudinally of the sheath to the distal end to define at least two leaf edges in the radially inner layer which are configured to move circumferentially with respect to each other to accommodate elastic radial expansion of the overlying radially outer layer as the sheath is retracted proximally with respect to the stent.

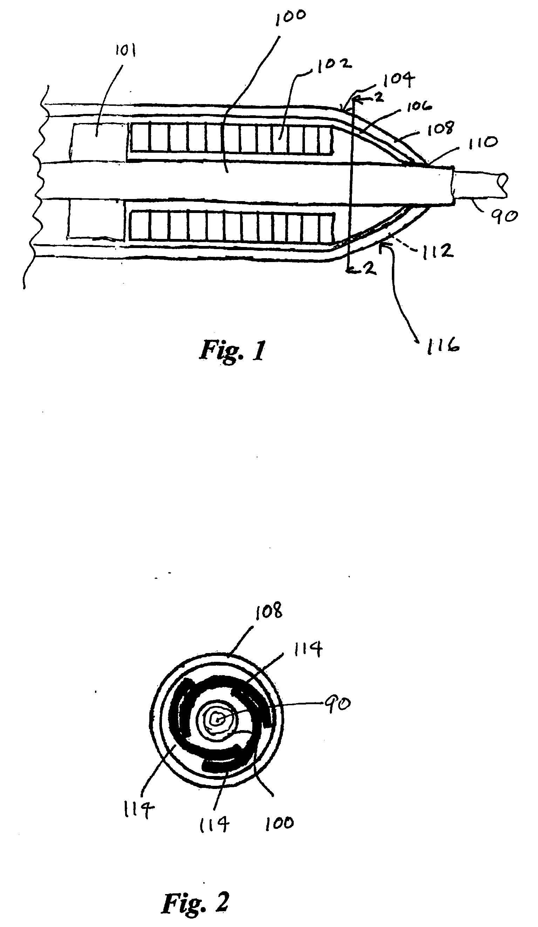

Referring to Figure 1, there is shown a inner catheter 100 providing a lumen to receive a guide wire 90. The inner catheter 100 carries, towards its distal end, a stent 102 which lies within a delivery sheath 104. In this figure, the stent 102 is illustrated diagrammatically. The precise form of the stent is not material to the present invention. Typically, the stent will be a self expanding stent capable of moving from the radially compressed form depicted in the figure to a radially expanded form, by virtue of the resilience of the stent or through shape memory behavior. The stent may be formed of a superelastic material, for example Nitinol.

Proximally of the stent 102, the inner catheter 100 carries a pusher ring 101 which is fixed relatively to the inner catheter 100 and serves to restrain movement of the stent 102 in a proximal direction, relative to the inner catheter 100.

The delivery sheath 104, in the distal region depicted in the figure, comprises a radially inner sheath layer 106 and a radially outer sheath layer 108. Both the radially inner sheath layer 106 and the radially outer sheath layer 108 extend to the distal end 110 of the sheath. The radially inner sheath layer 106 is in one example formed of PTFE .The radially outer sheath layer 108 is in one example formed of Nylon. Polyether Block Amide and Polyester Block Amide may also be suitable materials.

The radially inner sheath layer 106 has in the distal region three longitudinally extending slits 112, one of which is depicted in dotted line in Figure 1. As seen in Figure 2, these slits 112 divide the radially inner sheath layer 106 into three leaves 114. In the configuration shown in Figures 1 and 2, prior to deployment of the stent 102. The leaf edges of these three leaves 114 overlap circumferentially.

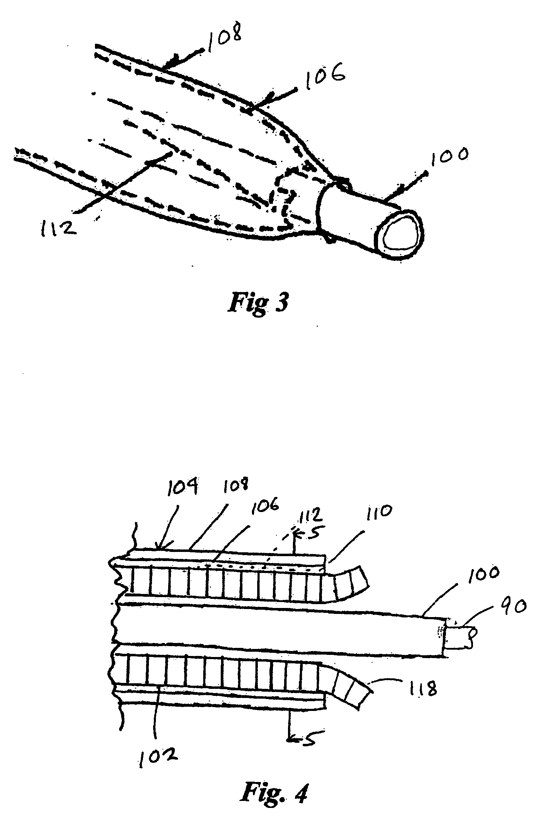

This overlapping of the leaves 114 of the radially inner sheath layer 106 is seen most clearly in Figure 3.

It will be seen that in the region 116 of the delivery sheath 104 that is distal of the stent 102, the delivery sheath tapers in the distal direction from a larger diameter to a smaller diameter. At the larger diameter, the delivery sheath has an internal diameter that is equal to or greater than the outer diameter of the stent 102. At the smaller diameter, the delivery sheath has an internal diameter that is less than the outer diameter of the stent 102 and which is equal to or greater than the outer diameter of the wire guide 100. It will be recognized that this distal region 116 of the delivery sheath provides, as the delivery sheath is advanced over the guide wire 100, an important atraumatic surface. Separately or in combination, the dilating surface may assist in the passage of the delivery sheath along the vessel, covering the distal extremity of the stent, preventing or reducing any risk of the distal extremity of the stent engaging with the vessel wall or hampering forward movement. This surface may in some applications also serve as a dilating surface, assisting in opening the vessel through which the delivery sheath is being advanced from an internal diameter greater than that of the wire guide to an internal diameter greater than that of the stent and that portion of the delivery sheath that surrounds the stent.

In use, the Seldinger technique is used to gain access and place the wire guide 90 which is maneuvered to the appropriate location in a vessel. , The delivery system, including inner catheter 100 and delivery sheath 104 is then introduced over the wire guide.

Once the distal end of the delivery system has been maneuvered to the correct location, the stent is deployed with proximal retraction of the delivery sheath 104 relative to the stent 102. A first stage in this proximal retraction is depicted in Figure 4, where the distal end 110 of the delivery sheath is shown retracted relative to the distal extremity of the stent 102. The beginning of the radial expansion of the stent is depicted at stent region 118.

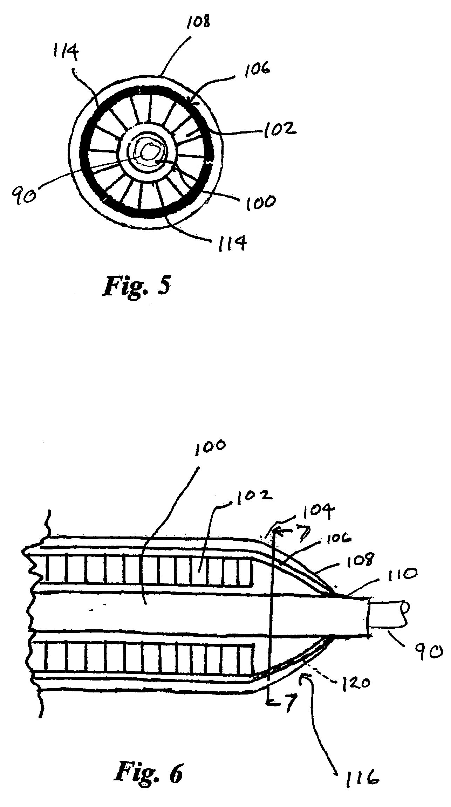

This retraction of the delivery sheath proximally over the stent is accompanied by a radial expansion of the elastic radially outer sheath layer 108. It is also accompanied by movement of the leaf edges of the radially inner sheath layer 106 circumferentially with respect to each other to accommodate the elastic radial expansion of the overlying radially outer layer. This is seen more clearly in Figure 5, which is a radial section on line 5-5 of the stent delivery system shown in Figure 4. The leaf edges have moved to the extent that the leaves 114 no longer overlap to any material extent. In this expanded configuration, the edges of the leaves may abut, may overlap to a slight extent and may be spaced circumferentially one from the other.

It will be seen that in this initial retraction and as the delivery sheath continues to be retracted proximally of the stent, substantially the only contact between the delivery sheath and the stent occurs at the low friction inner layer 106. This considerably assists in the retraction. The risk of the retracting delivery sheath catching the stent, particularly acute if the stent is to be deployed in a curved region of the vascular system, is significantly reduced. Catching of the stent by the retracting delivery sheath - if it is not avoided - can lead to positional errors in the deployment of the sheath and damage to the vascular system.

The tapering of the delivery sheath at the distal end may be achieved by dip coating of an appropriately shaped mandrel, in a molding process or using other appropriate techniques.

The wall thickness of the radially outer sheath layer 108 in the distal tapered region may itself taper, reducing in a direction toward the distal end. This is depicted in Figure 6. The wall thickness of the radially outer sheath layer 108 may reduce so that at the distal end of the sheath the wall thickness is from 10% to 90% (or 30% to 70%) of the wall thickness of the radially outer sheath layer 108 in the region of the delivery sheath overlying the stent 102. In this way, or in other ways, it is provided that the resistance of the outer layer to hoop stresses decreases towards the distal end. That is to say the resistance to radial expansion reduces as the extent of required radial expansion increases.

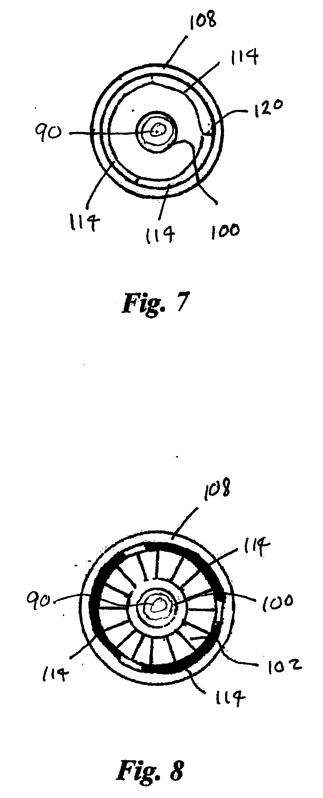

In one modification, the described slits 112 in the radially inner sheath layer 106 are each replaced by a line of weakness. This is depicted in Figure 7, where the leaves 114 are essentially continuous, separated only by the lines of weakness 120. As the distal region of the delivery sheath expands radially to accommodate retraction of the delivery sheath proximally over the stent, the leaves 114 separate at the lines of weakness 120 to adopt the configuration shown in Figure 8. In this movement, the neighboring leaf edges move circumferentially away from each other, rather than circumferentially toward each other as in the previous described example. This arrangement has the feature that some significant regions of the radially outer sheath layer 108 are exposed to the stent 102, and is for this reason in some cases not preferred.

In a further modification, the inner catheter of stent delivery system may also include a stent pulling formation disposed distally of the stent, enabling the stent to be moved proximally, in adjusting the stent location prior to deployment. For more detail of such an inner catheter in a stent delivery system see published patent application US 2006/0259117.

Whilst the described examples have referred to stents in the vascular system, it will be apparent that the invention may relate also to the delivery of stents in other bodily vessels (for example biliary applications) and to the delivery of other endoluminal prostheses, including grafts, filters and valves. Similarly, the present invention may have application in arrangements not using a wire guide.

Still further modifications may be made without departing from the scope of the appended claims.

## Claims

1. A delivery sheath for delivering stents or other endoluminal prostheses, the sheath having a distal end with a radially inner sheath layer and a radially outer sheath layer both extending to the distal end of the sheath, wherein the radially inner layer has at least one separation line extending longitudinally of the sheath to the distal end to define at least two leaf edges in the radially inner layer,
**characterised in that**,
the radially inner layer comprises a material having a low coefficient of friction and the radially outer layer comprises an elastic material and at least two leaf edges in the radially inner layer are configured to move circumferentially with respect to each other to accommodate elastic radial expansion of the overlying radially outer layer.

2. A delivery sheath according to Claim 1, wherein the radially inner layer has at least two separation lines extending longitudinally of the sheath to the distal end to define at least two leaves which are configured to move circumferentially with respect to each other to accommodate elastic radial expansion of the overlying radially outer layer.

3. A delivery sheath according to Claim 1 or Claim 2, wherein the separation line or each separation line is a line of weakness in the radially inner layer.

4. A delivery sheath according to Claim 1 or Claim 2, wherein one leaf edge overlies another leaf edge so that the leaf edges move circumferentially toward each other to accommodate elastic radial expansion of the overlying radially outer layer.

5. A delivery sheath according to any one of the preceding claims in which the outer diameter of the delivery sheath tapers with the diameter reducing in a direction toward the distal end.

6. A delivery sheath according to any one of the preceding claims in which the radially outer layer is formed of a thermoplastic polymer material.

7. A delivery sheath according to any one of the preceding claims in which the radially outer layer is formed of a material selected from the group consisting of Nylon, Polyether Block Amide and Polyester Block Amide.

8. A delivery sheath according to any one of the preceding claims in which the radially inner layer is formed of PTFE.

9. A delivery sheath according to any one of the preceding claims in which the resistance of the outer layer to hoop stresses decreases towards the distal end.

10. A delivery system, comprising a delivery sheath in accordance with any of the preceding claims and an endoluminal prosthesis located within the delivery sheath in the distal portion so that the distal portion of the delivery sheath extends circumferentially around the prosthesis, the delivery sheath tapering distally of the prosthesis to a reduced diameter at the distal end of the sheath so providing an atraumatic surface distally of the prosthesis,
said elastic radial expansion of the overlying radially outer layer occurring as the sheath is retracted proximally with respect to the prosthesis.

11. A delivery system according to Claim 10, comprising an inner catheter disposed coaxially within the delivery sheath, the endoluminal prosthesis being carried on said inner catheter.

12. A delivery system according to Claim 11, wherein the inner catheter includes a radially extending formation for engagement with the endoluminal prosthesis.

## Patentansprüche

1. Zuführungshülse zum Zuführen von Stents oder anderen endoluminalen Prothesen, wobei die Hülse ein distales Ende mit einer radial inneren Hülsenschicht und einer radial äußeren Hülsenschicht hat, die sich beide zum distalen Ende der Hülse erstrecken, wobei die radial innere Schicht mindestens eine Trennlinie hat, die sich in Längsrichtung der Hülse zum distalen Ende erstreckt, um mindestens zwei Blattränder in der radial inneren Schicht zu definieren,
**dadurch gekennzeichnet, dass**
die radial innere Schicht ein Material mit einem niedrigen Reibungskoeffizienten umfasst und die radial äußere Schicht ein elastisches Material umfasst und mindestens zwei Blattränder in der radial inneren Schicht dazu konfiguriert sind, sich umfangsmäßig bezüglich einander zu bewegen, um elastische radiale Ausdehnung der darüberliegenden radial äußeren Schicht aufzunehmen.

2. Zuführungshülse nach Anspruch 1, wobei die radial innere Schicht mindestens zwei Trennlinien hat, die sich in Längsrichtung der Hülse zum distalen Ende erstrecken, um mindestens zwei Blätter zu definieren, die dazu konfiguriert sind, sich umfangsmäßig bezüglich einander zu bewegen, um elastische radiale Ausdehnung der darüberliegenden radial äußeren Schicht aufzunehmen.

3. Zuführungshülse nach Anspruch 1 oder Anspruch 2, wobei die Trennlinie oder jede Trennlinie eine Schwächungslinie in der radial inneren Schicht ist.

4. Zuführungshülse nach Anspruch 1 oder Anspruch 2, wobei ein Blattrand über einem anderen Blattrand liegt, so dass sich die Blattränder umfangsmäßig aufeinander zubewegen, um elastische radiale Ausdehnung der darüberliegenden radial äußeren Schicht aufzunehmen.

5. Zuführungshülse nach einem der vorhergehenden Ansprüche, wobei sich der äußere Durchmesser der Zuführungshülse verjüngt, wobei der Durchmesser in einer Richtung zum distalen Ende hin abnimmt.

6. Zuführungshülse nach einem der vorhergehenden Ansprüche, wobei die radial äußere Schicht aus einem thermoplastischen Polymermaterial gebildet ist.

7. Zuführungshülse nach einem der vorhergehenden Ansprüche, wobei die radial äußere Schicht aus einem Material gebildet ist, das aus der aus Nylon, Polyetherblockamid und Polyesterblockamid bestehenden Gruppe ausgewählt ist.

8. Zuführungshülse nach einem der vorhergehenden Ansprüche, wobei die radial innere Schicht aus PTFE gebildet ist.

9. Zuführungshülse nach einem der vorhergehenden Ansprüche, wobei die Umfangsspannungsbeständigkeit der äußeren Schicht zum distalen Ende hin abnimmt.

10. Zuführungssystem, umfassend eine Zuführungshülse nach einem der vorhergehenden Ansprüche und eine endoluminale Prothese, die im distalen Abschnitt in der Zuführungshülse angeordnet ist, so dass sich der distale Abschnitt der Zuführungshülse umfangsmäßig um die Prothese herum erstreckt, wobei sich die Zuführungshülse distal zu der Prothese auf einen verringerten Durchmesser am distalen Ende der Hülse verjüngt, so dass distal zu der Prothese eine atraumatische Oberfläche bereitgestellt ist,
wobei die elastische radiale Ausdehnung der darüberliegenden radial äußeren Schicht erfolgt, wenn die Hülse proximal bezüglich der Prothese zurückgezogen wird.

11. Zuführungssystem nach Anspruch 10, umfassend einen inneren Katheter, der koaxial in der Zuführungshülse angeordnet ist, wobei die endoluminale Prothese auf dem inneren Katheter getragen wird.

12. Zuführungssystem nach Anspruch 11, wobei der innere Katheter ein sich radial erstreckendes Gebilde zur Ineingriffnahme der endoluminalen Prothese aufweist.

## Revendications

1. Gaine de pose pour mettre en place des stents ou autres endoprothèses, la gaine comportant une extrémité distale avec une couche radialement intérieure de gaine et une couche radialement extérieure de gaine s'étendant toutes les deux jusqu'à l'extrémité distale de la gaine,
dans laquelle la couche radialement intérieure comporte au moins une ligne de séparation s'étendant le long de la gaine jusqu'à son extrémité distale pour définir au moins deux bords de feuille dans la couche radialement intérieure,
**caractérisée en ce que** la couche radialement intérieure comprend une matière ayant un faible coefficient de frottement et la couche radialement extérieure comprend une matière élastique et qu'au moins deux bords de feuille dans la couche radialement intérieure sont configurés pour se déplacer l'un par rapport à l'autre suivant la circonférence pour s'adapter à la dilatation radiale élastique de la couche radialement extérieure superposée.

2. Gaine de pose selon la revendication 1, dans laquelle la couche radialement intérieure comporte au moins deux lignes de séparation s'étendant le long de la gaine jusqu'à son extrémité distale pour définir au moins deux feuilles qui sont configurées pour se déplacer l'une par rapport à l'autre suivant la circonférence pour s'adapter à la dilatation radiale élastique de la couche radialement extérieure superposée.

3. Gaine de pose selon la revendication 1 ou 2, dans laquelle la ligne de séparation ou chaque ligne de séparation est une ligne de faiblesse dans la couche radialement intérieure.

4. Gaine de pose selon la revendication 1 ou 2, dans laquelle un bord de feuille recouvre un autre bord de feuille de façon à ce que les bords de feuille se déplacent l'un vers l'autre suivant la circonférence pour s'adapter à la dilatation radiale élastique de la couche radialement extérieure superposée.

5. Gaine de pose selon l'une quelconque des revendications précédentes, dans laquelle le diamètre extérieur de la gaine de pose diminue progressivement, le diamètre se réduisant dans la direction de l'extrémité distale.

6. Gaine de pose selon l'une quelconque des revendications précédentes, dans laquelle la couche radialement extérieure est constituée d'une matière polymère thermoplastique.

7. Gaine de pose selon l'une quelconque des revendications précédentes, dans laquelle la couche radialement extérieure est constituée d'une matière choisie dans le groupe rassemblant le nylon, les copolymères amide-polyéther et les copolymères amidepolyester.

8. Gaine de pose selon l'une quelconque des revendications précédentes, dans laquelle la couche radialement intérieure est constituée de PTFE.

9. Gaine de pose selon l'une quelconque des revendications précédentes, dans laquelle la résistance de la couche extérieure aux efforts circonférentiels décroît vers l'extrémité distale.

10. Système de mise en place, comprenant une gaine de pose selon l'une quelconque des revendications précédentes et une endoprothèse située à l'intérieur de la gaine de pose dans sa partie distale de façon à ce que la partie distale de la gaine de pose s'étende autour de la circonférence de la prothèse, la gaine de pose s'effilant en direction distale de la prothèse jusqu'à un diamètre réduit à l'extrémité distale de la gaine, offrant ainsi une surface non traumatisante dans la direction distale de la prothèse,
ladite dilatation radiale élastique de la couche radialement extérieure superposée intervenant lorsqu'on retire la gaine dans la direction proximale par rapport à la prothèse.

11. Système de mise en place selon la revendication 10, comprenant un cathéter intérieur disposé coaxialement à l'intérieur de la gaine de pose, l'endoprothèse étant transportée sur ledit cathéter intérieur.

12. Système de mise en place selon la revendication 11, dans lequel le cathéter intérieur comprend une formation à extension radiale pour entrer en prise avec l'endoprothèse.
